# FASCICULE DE BREVET EUROPEEN

(11) **EP 3 773 436 B1**
(45) Date de publication et mention de la délivrance du brevet: **01.06.2022**
(21) Numéro de dépôt: 19721002.4
(22) Date de dépôt: 09.04.2019
(51) Int. Cl.: A61Q 19/02, A61K 8/34, A61K 8/60, A61K 8/49, A61K 31/047, A61K 31/34, A61K 31/7004, A61K 31/715

(54) **UTILISATION DE SORBITYL POLYRHAMNOSIDES COMME AGENTS ÉCLAIRCISSANTS DE LA PEAU HUMAINE**
VERWENDUNG VON SORBITYLPOLYRHAMNOSIDEN ALS AUFHELLER DER MENSCHLICHEN HAUT
USE OF SORBITYL POLYRHAMNOSIDES AS HUMAN SKIN LIGHTENING AGENTS

(30) Priorité: 11.04.2018 FR 1853146
(43) Date de publication de la demande: 17.02.2021
(73) Titulaire: Société d'Exploitation de Produits pour les Industries Chimiques SEPPIC, 75321 Paris cedex 07 (FR)
(72) Inventeur: CATTUZZATO, Laetitia, 81100 CASTRES (FR); DACOSTA, Georges, 81710 SAIX (FR); DESSILLA, Stéphane, 81100 CASTRES (FR); GUILBOT, Jérôme, 81100 CASTRES (FR)
(74) Mandataire: Air Liquide
(86) Numéro de dépôt international: PCT/FR2019/050831
(87) Numéro de publication internationale: WO 2019/197774

(56) Documents cités:
- EP-A1- 3 196 204
- WO-A2-2010/034917
- CN-A- 102 908 278
- FR-A1- 3 027 517
- US-A1- 2007 128 128
- VINCE POZSGAY ET AL: "Synthetic oligosaccharides related to group B streptococcal polysaccharides. 3. Synthesis of oligosaccharides corresponding to the common polysaccharide antigen of group B streptococci", JOURNAL OF ORGANIC CHEMISTRY, vol. 53, no. 17, 1 août 1988 (1988-08-01), pages 4042-4052, XP055529883, ISSN: 0022-3263, DOI: 10.1021/jo00252a030

## Description

La présente invention a pour objet l'utilisation d'une composition obtenue par réaction de rhamnose et de sorbitol comme agent d'éclaircissement de la peau du corps humain, ainsi que les procédés non thérapeutiques d'éclaircissement de la peau humaine mettant en oeuvre une formulation cosmétique en comprenant.

La peau humaine constitue la première image offerte au regard d'autrui, et par conséquent, l'amélioration de son aspect est un sujet de préoccupation constant pour les êtres humains. La peau est le reflet d'un état de bien-être, souvent associé à la jeunesse, et a *contrario* d'un état de fatigue et/ou de vieillissement. Si dans de nombreux pays les consommateurs cherchent à brunir la peau, soit naturellement sous l'action des rayonnements UV du soleil soit artificiellement sous l'action de composés chimiques, certains consommateurs recherchent à éclaircir leur peau, pour procurer une image externe de pureté, ou dans certains pays d'Asie ou d'Afrique pour procurer une image de beauté. Pour les peaux caucasiennes, le besoin d'éclaircissement peut également s'inscrire dans une volonté de masquer l'apparition de tâches pigmentaires (dites « tâches de sénescence ») liées à l'âge.

La couleur et l'éclat du teint de la peau résultent de la combinaison de deux composantes biologiques : la vascularisation et la pigmentation de la peau ; ladite pigmentation de la peau étant la résultante des quantités de mélanines et de carotènes présentes dans la peau humaine.

Les mélanines sont produites par des cellules épidermiques spécifiques : les mélanocytes. Ceux-ci se retrouvent au niveau de la couche basale de l'épiderme ou dans les follicules pileux. Les mélanines sont des cellules de grande taille, présentant de nombreux et longs prolongements que l'on nomme dendrites. Ces dendrites entrent en contact avec les kératinocytes environnants, pouvant se situer jusqu'à deux assises cellulaires supérieures, pour transmettre les mélanines regroupées dans des mélanosomes.

On désigne par Unité Epidermique de Mélanisation (UEM), l'ensemble composé des mélanocytes et des kératinocytes, interagissant ensemble pour donner la couleur à la peau. Une UEM comprend notamment un mélanocyte et une quarantaine de kératinocytes. Le processus de mélanogenèse en lui-même comprend la synthèse des mélanines par les mélanocytes, leur transfert et leur distribution aux kératinocytes avoisinants.

La synthèse de mélanines se produit dans les mélanocytes, et plus particulièrement dans des organites spécialisés, les mélanosomes. Cette synthèse se fait à partir de la tyrosine et fait intervenir plusieurs enzymes, dont la tyrosinase qui est la première des enzymes à intervenir dans la chaîne réactionnelle.

La pigmentation de la peau peut soit être constitutive soit induite. La pigmentation constitutive repose sur le patrimoine génétique de l'individu, les facteurs hormonaux et l'âge.

La pigmentation induite résulte principalement de l'expositions aux rayonnements ultraviolets. Elle apparaît entre 48 heures et 72 heures après ladite exposition et disparaît rapidement après. Elle résulte notamment de l'activation de la tyrosinase, d'une augmentation de la taille et du nombre de mélanocytes et d'un accroissement du transfert des mélanosomes aux kératinocytes. Une action indirecte sur les kératinocytes entre également en jeu pour favoriser la mélanogénèse par la sécrétion de facteurs solubles. Au contraire, une inhibition de l'activité de la tyrosinase induira une diminution de la quantité des mélanines produites par les mélanocytes et conduira à sa manifestation visible, à savoir l'éclaircissement naturel de la peau humaine.

De nombreuses formulations cosmétiques comprennent comme agents éclaircissants de la peau l'acide kojique, l'arbutine et/ou le magnésium ascorbyl phosphate.

L'hydroquinone, substance historiquement utilisée pour éclaircir la peau, est maintenant interdite dans certains pays du fait de son action altérante de la peau et des dangers qu'elle induit pour la santé du consommateur.

La demande de brevet européen publiée sous le numéro EP 1 471 881 divulgue que des dérivés N-acylés d'acides alpha-aminés et notamment le N-undécylénoyl phénylalanine, présentent une affinité vis à vis du récepteur de la Mélanocyte Spécifique Hormone (α-MSH) et induisent ainsi l'éclaircissement de la peau selon le mécanisme biochimique suivant : la compétition entre l'hormone α-MSH et la molécule présentant une affinité vis à vis du récepteur α-MSH, entraîne un moindre taux de fixation de ladite hormone sur les récepteurs cellulaires ; cette compétition a pour conséquence une inhibition de l'activité de l'adénylate cyclase qui entraîne une moindre transformation de l'ATP en AMP cyclique intracellulaire ; la diminution du taux d'AMP cyclique entraîne une inhibition de l'enzyme Protéine Kinase A (PKA) ; l'inhibition de la Protéine Kinase A induit une moindre activation de la tyrosinase du fait de la moindre transformation de cette dernière en tyrosinase phosphorylée ; cette moindre activation de la tyrosinase entraîne la diminution de la synthèse de mélanine d'où découle une moindre pigmentation de la peau.

La demande de brevet japonais N° 2000-229121 décrit l'utilisation d'esters de N-acyl aminoacides et de polyols comme tensioactifs performants

La demande internationale publiée sous le numéro WO 2010/034917 décrit les mono-esters et les di-esters de polyols du N-(ω-undécylènoyl) phénylalanine, et plus particulièrement les mono-esters et les di-esters résultant de la réaction du glycérol et du N-(ω-undécylènoyl) phénylalanine, et leurs utilisations comme agents éclaircissants de la peau humaine.

La demande internationale de brevet publiée sous le numéro WO 2013/001192 A1 décrit des mono-esters et des di-esters résultant de la réaction d'estérification entre de l'isosorbide et des dérivés N-acylés d'acides aminés, leurs utilisations comme agent actif cosmétique, pour prévenir et/ou limiter les effets inesthétiques générés par l'hypoxie des cellules endothéliales du corps humain, et plus particulièrement pour prévenir et/ou limiter les effets inesthétiques générés par les cernes, les poches péri-oculaires et/ou les jambes lourdes.

Certains dérivés du glycérol, en particulier les acétals de glycérol, résultant de la condensation avec un sucre réducteur sont utilisés comme agents hydratants de la peau humaine. On peut citer notamment :
- Les acétals issus de la condensation du glycérol avec le glucose, qui sont divulgués dans la demande de brevet européen publiée sous le numéro EP 0 770 378 ; ils présentent effectivement une meilleure tolérance cutanée que le glycérol mais ont un pouvoir hydratant généralement plus faible ; et
- Le mannosyl érythritol décrit dans la demande de brevet japonais publiée sous le numéro JP 63063390 A2 qui est obtenu selon un procédé de préparation par voie enzymatique, et connu pour son activité de rétention de l'hydratation cutanée.

Parmi les acétals du glucose et d'autres polyols, on peut citer les glycosides obtenus par acétalisation de l'érythritol, du xylitol ou du sorbitol, tels que décrits dans la demande de brevet français publiée sous le numéro FR 2 839 447 A1. Ces composés ont démontré de meilleures propriétés hydratantes que les produits d'acétalisation du glycérol susmentionné.

Les polyols polyglycosides (ou PPG) sont obtenus de la même manière que les alkylpolyglucosides (ou APG) à partir de glucose et en remplaçant les alcools gras de départ par des polyols. A la fin des réactions de glycosylation, les produits peuvent être commercialisés tels quels en solution dans le polyol de départ ou sous forme de solutions aqueuses. De manière similaire aux APG, les PPG ciblés se caractérisent par un degré de polymérisation moyen (DP) très proche de 1.

Seuls des polyols polyglucosides sont disponibles commercialement, à savoir le glycérol polyglucoside sous le nom de marque Hydragen^{™}GG et une composition comprenant du xylityl polyglucoside sous le nom de marque Aquaxyl^{™}.

Dans le cadre de leurs recherches de nouveaux actifs éclaircissant la peau, améliorés par rapport à ceux de l'état de la technique cités ci-dessus, les inventeurs ont développé une nouvelle solution technique consistant en l'utilisation de produits résultant de la réaction entre le rhamnose et le sorbitol.

Ainsi, selon un premier aspect, l'invention a pour objet l'utilisation non thérapeutique d'une composition (C₁) comprenant pour 100% de sa masse :
(1) - Une quantité supérieure à 0% en masse et inférieure ou égale à 25% en masse d'un polyol de formule (I) :

   HO-CH₂-(CHOH)₄-CH₂-OH (I) ;
(2) - Une quantité supérieure à 0% en masse et inférieure ou égale à 30% en masse d'un composé de formule (B₁₃) : 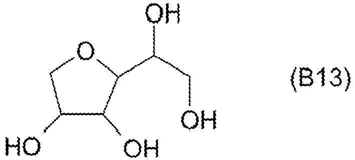
(3) - Une quantité supérieure ou égale à 0% en masse et inférieure ou égale à 5% en masse d'un composé de formule (B₁₄) : 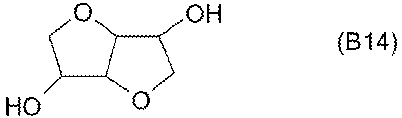
(4) - De 60% en masse à 95% en masse d'une composition (C_{R}) représentée par la formule (II) :

   HO-CH₂CHOH)₄-CH₂-O-(Rham)ₓ-H (II),

   formule (II) dans laquelle Rham représente le reste du rhamnose et x, qui indique le degré moyen de polymérisation dudit reste Rham, représente un nombre décimal supérieur ou égal à 1,5 et inférieur ou égal à 2,5, et
(5) - une quantité supérieure à 0% en masse et inférieure ou égale à 5% en masse de rhamnose ;
   étant entendu que la somme des proportions massiques en composés ou composition selon (1), (2), (3), (4) et (5) est égale à 100% massique,
   ladite utilisation étant dans le but d'éclaircir la peau humaine, et ladite utilisation étant dans une composition cosmétique.

Par la formule (B₁₃) on représente dans la définition précédente, le composé résultant de l'anhydrisation, selon une réaction d'intra-cyclisation du composé de formule (I), et par la formule (B₁₄), le composé résultant de l'anhydrisation, selon une réaction d'intra-cyclisation dudit composé de formule (B₁₃).

Par la formule (II) HO-CH₂-(CHOH)₄-CH₂-O-(Rham)ₓ-H, on signifie que ladite composition (C_{R}) consiste essentiellement en un mélange de composés représentés par les formules (II₁), (II₂), (II₃), (II₄) et (II₅) :

HO-CH₂-(CHOH)₄-CH₂-O-(Rham)₁-H (II₁),

HO-CH₂-(CHOH)₄-CH₂-O-(Rham)₂-H (II₂),

HO-CH₂-(CHOH)₄-CH₂-O-( Rham)₃-H (II₃),

HO-CH₂-(CHOH)₄-CH₂-O-( Rham)₄-H (II₄),

HO-CH₂-(CHOH)₄-CH₂-O-( Rham)₅-H (II₅),

dans les proportions molaires respectives a₁, a2, a3, a4 et a₅, telles que la somme a₁+a₂+a₃+a₄+a₅ est égale à 1 et que la somme a₁+2a₂+3a₃+4a₄+5a₅ est égale à x.

Par essentiellement, on indique dans la définition qui précède, que la présence d'un ou de plusieurs composés de formule (II_{w}) avec w supérieur à 5 n'est pas exclue au sein de la composition (C_{R}), mais que si présence il y a, elle l'est en des proportions minimes qui n'entraînent aucune modification substantielle des propriétés de ladite composition (C_{R}).

Dans la formule (II) telle que définie ci-dessus, le groupe HO-CH₂-(CHOH)a-CH₂-O- est lié à (Rham)ₓ par le carbone anomérique du reste saccharide, de manière à former une fonction acétal.

La composition (C₁) telle que définie précédemment peut être préparée selon un procédé comprenant les étapes successives suivantes :
- Une **étape a)** de chauffage sous agitation douce du polyol de formule (I) telle que définie précédemment, jusqu'à le porter à une température supérieure (T₁) d'au moins 5°C au-dessus de sa température de fusion, soit une température (T₁) supérieure ou égale à 95°C et inférieure ou égale à 135°C et plus particulièrement supérieure ou égale à 110°C et inférieure ou égale à 135°C ;
- Une **étape b)** de dispersion sous agitation de rhamnose mono-hydraté dans le milieu précédemment fondu dans le rapport stœchiométrique souhaité ;
- Une **étape c)** d'acétalisation en ajoutant sous agitation dans le milieu liquide issu de l'étape b) une quantité catalytique d'acide protique, en maintenant le mélange réactionnel sous vide partiel et en distillant l'eau formée ; et si nécessaire ou si désiré,
- Une **étape d)** de neutralisation de la solution obtenue à l'issue de l'étape c), pour obtenir ladite composition (C₁).

Selon une première alternative du procédé tel que défini précédemment, dans la mise en œuvre de l'étape a), le sorbitan de formule (B13) est utilisé à la place du sorbitol.

Pour la mise en œuvre de l'étape c) telle que définie ci-dessus, l'acide protique est notamment choisi parmi les acides sulfurique, chlorhydrique, phosphorique, nitrique, hypophosphoreux, méthane-sulfonique, para- toluène sulfonique, trifluorométhane sulfonique et les résines échangeuses d'ions acides. Habituellement, la réaction d'acétalisation est effectuée à une température (T₂) de supérieure ou égale à 70°C et inférieure ou égale à 135°C, sous un vide supérieur ou égal à 300 10² Pa et inférieur ou égal à 20 10² Pa (300 à 20 mbar).

L'invention a aussi pour objet un procédé non thérapeutique dans le but d'éclaircir la peau humaine comprenant au moins une étape d'application sur ladite peau humaine d'une formulation cosmétique à usage topique comprenant au moins un excipient cosmétiquement acceptable et une quantité efficace de la composition (C₁) telle que définie précédemment.

L'expression "à usage topique" utilisée dans la définition de la formulation cosmétique mise en oeuvre dans le procédé cosmétique objet de la présente invention, signifie que ladite formulation est mise en oeuvre par application sur la peau, qu'il s'agisse d'une application directe dans le cas d'une formulation cosmétique ou d'une application indirecte lorsque la formulation cosmétique selon l'invention est imprégnée sur un support destiné à être mis en contact avec la peau (papier, lingette, textile, dispositif transdermique, etc...).

L'expression « cosmétiquement acceptable » utilisée dans la définition de la formulation cosmétique à usage topique, mise en oeuvre dans le procédé cosmétique objet de la présente invention, signifie selon la directive du Conseil de la Communauté Economique Européenne N°76/768/CEE du 27 juillet 1976 modifiée par la directive N°93/35/CEE du 14 juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par quantité efficace d'une composition (C₁) telle que définie précédemment et présente dans la formulation cosmétique à usage topique mise en oeuvre dans le procédé cosmétique objet de la présente invention, on entend pour 100% de la masse de ladite formulation cosmétique à usage topique, une proportion comprise entre 0,01% et 5% massique, plus particulièrement entre 0,01% et 3% massique, encore plus particulièrement entre 0,1% et 3% massique, et encore plus particulièrement entre 0,5% et 2% massique en composition (C₁).

Les formulations cosmétiques à usage topique mises en oeuvre dans le procédé cosmétique objet de la présente invention, se présentent généralement sous forme de solutions aqueuses ou hydro-alcooliques ou hydro-glycoliques, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

Les formulations cosmétiques à usage topique mises en oeuvre dans le procédé cosmétique objet de la présente invention, peuvent être conditionnées dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, la composition (C₁), présente dans les formulations cosmétiques à usage topique mises en oeuvre dans le procédé cosmétique objet de la présente invention, est associée à des additifs chimiques habituellement mis en œuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer à ladite composition (C₁), on peut citer les tensioactifs moussants et/ou détergents anioniques, cationiques, amphotères ou non ioniques ;
- parmi les tensioactifs anioniques moussants et/ou détergents, il y a par exemple les sels de métaux alcalins, de métaux alcalino-terreux, d'ammonium, d'amines, ou d'aminoalcools d'alkylethersulfates, d'alkylsulfates, d'alkylamidoéthersulfates, d'alkylarylpolyéthersulfates, de monoglycérides sulfates, d'alpha-oléfinesulfonates, de paraffinessulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, de dérivés N-acylés d'acides gras.
- parmi les tensioactifs amphotères moussants et/ou détergents, il y a par exemple les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.
- parmi les tensioactifs cationiques moussants et/ou détergents, il y a par exemple les dérivés d'ammoniums quaternaires.
- parmi les tensioactifs non ioniques moussants, il y a par exemple les alkyl polyglycosides comportant un radical aliphatique, linéaire ou ramifié, saturé ou insaturé, et comportant de huit à seize atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1,12-dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à ladite composition (C₁), il y a les esters gras d'alkyl polyglycosides éventuellement alcoxylés, comme les esters de méthyl polyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ; les esters gras alcoxylés tels que le PEG 150 pentaérythrytyl tétrastéarate commercialisé sous l'appellation CROTHIX^{™} DS53, le PEG 55 propylène glycol oléate commercialisé sous l'appellation ANTIL^{™} 141 ; les carbamates de polyalkylène glycols à chaînes grasses comme le PPG-14 laureth isophoryl dicarbamate commercialisé sous l'appellation ELFACOS^{™} T211, le PPG-14 palmeth-60 hexyl dicarbamate commercialisé sous l'appellation ELFACOS^{™} GT2125.

Comme exemples d'agents épaississants et/ou gélifiants que l'on peut associer à ladite composition (C₁), il y a les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide-2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

CH₂=C(R₃)-C(=O)-[CH₂-CH₂-O]ₙ-R₄ (VIII)

dans laquelle R₃ représente un atome d'hydrogène ou un radical méthyle, R4 représente un radical alkyle linéaire ou ramifié comportant de huit à trente atomes de carbone et n représente un nombre supérieur ou égal à un et inférieur ou égal à cinquante.

Lesdits polymères peuvent se présenter sous la forme d'une solution, d'une suspension aqueuse, d'une émulsion eau-dans-huile, d'une émulsion huile-dans-eau, d'une poudre. Comme exemples de polymères du commerce, il y a ceux commercialisés sous les appellations SIMULGEL^{™}EG, SIMULGEL^{™}EPG, SEPIGEL^{™}305, SIMULGEL^{™}600, SIMULGEL^{™}NS, SIMULGEL^{™}INS100, SIMULGEL^{™}FL, SIMULGEL^{™}A, SIMULGEL^{™}SMS 88, SEPINOV^{™}EMT10, SEPIPLUS^{™}400, SEPIPLUS^{™}265, SEPIPLUS^{™}S, SEPIMAX^{™}Zen, ARISTOFLEX^{™}AVC, ARISTOFLEX^{™}AVS, NOVEMER^{™}EC-1, NOVEMER^{™}EC2, ARISTOFLEX^{™}HMB, COSMEDIA^{™}SP, FLOCARE^{™}ET25, FLOCARE^{™}ET75, FLOCARE^{™}ET26, FLOCARE^{™}ET30, FLOCARE^{™}ET58, FLOCARE^{™}PSD30, VISCOLAM^{™}AT64, VISCOLAM^{™}AT100.

Comme autres exemples d'agents épaississants et/ou gélifiants que l'on peut associer à ladite composition (C₁), il y a :
- Les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, tels que les galactomannanes provenant de la gomme de cassia (DS = 1/5), de la gomme de caroube (DS = 1/4), de la gomme de tara (DS = 1/3), de la gomme de guar (DS = 1/2) ou de la gomme de fenugrec (DS = 1 ) ;
- Les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme arabique et de gomme de karaya, les glucosaminoglycanes ;
- La cellulose et ses dérivés la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à ladite composition (C₁), il y a les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples d'eaux thermales ou minérales que l'on peut associer à ladite composition (C₁), il y a les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à ladite composition (C₁), il y a les xylènes sulfonates, les cumènes sulfonates, l'hexyl polyglucoside, le (2-éthyl hexyl) polyglucoside, le n-heptyl polyglucoside.

Comme exemples d'agents déodorants que l'on peut associer à ladite composition (C₁), il y a les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de solvants et de co-solvants que l'on peut associer à ladite composition (C₁), il y a l'eau, l'éthylène glycol, le propylène glycol, le butylène glycol, l'hexylène glycol, le diéthylène glycol, les alcools hydrosolubles tels que l'éthanol, l'isopropanol ou le butanol, les mélanges d'eau et desdits solvants.

Comme exemples d'huiles que l'on peut associer à ladite composition (C₁), il y a les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les iso-alcanes de synthèse comme l'isohexadécane, l'isododécane, les huiles perfluorées ; les huiles de silicone comme les diméthylpolysiloxanes, les méthylphényl - polysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à ladite composition (C₁), il y a la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de matières grasses que l'on peut associer à ladite composition (C₁), il y a les alcools gras saturés ou insaturés, linéaires ou ramifiés, comportant de huit à trente-six atomes de carbone, ou les acides gras saturés ou insaturés, linéaires ou ramifiés, comportant de huit à trente-six 36 atomes de carbone.

Comme exemples de filtres solaires que l'on peut associer à ladite composition (C₁), il y a tous ceux figurant dans la directive cosmétique 76/768/CEE modifiée annexe VII : notamment les composés suivants :
- le 2-phényl 5-méthyl benzoxazole, le 2,2'-hydroxy-5-méthyl phényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des polysiloxanes comme le malonate de benzylidène siloxane ; ou ceux des familles de composés suivantes :
   - Famille l'acide benzoïque comme les acides para-amino benzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
   - Famille de l'acide anthranilique comme l'homomenthyl-N-acétyl anthranilate ;
   - Famille l'acide salicylique comme le salicylate d'amyle, le salicylate d'homomenthyle, le salicylate d'éthyl hexyle, le salicylate de phényle, le salicylate de benzyle, le salicylate de p-isopropyl phényle ;
   - Famille l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ;
   - Famille de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 4'-phényl benzophénone-2-carboxylate de (2-éthyl hexyle), la 2-hydroxy 4-(octyloxy) benzophénone, la 4-hydroxy benzophénone-3-carboxylique ; le 3-(4'-méthylbenzylidène)-d,l-camphre, le 3-(benzylidène)-d,l-camphre, le benzalkonium méthosulfate camphre ;
   - Famille de l'acide urocanique, comme cet acide ou l'urocanate d'éthyle ;
   - Famille de l'acide sulfonique comme l'acide 2-phényl benzimidazole-5-sulfonique et ses sels ;
   - Famille de la triazine comme l'hydroxyphényl triazine, l'(éthyl hexyloxy) (hydroxy) phényl 4-méthoxyphényl triazine, le 2,4,6-trianillino-(p-carbo-2'-éthyl hexyl-1'-oxy)-1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque ;
   - Famille du diphénylacrylate comme le 2-éthylhexyl-2-cyano-3,3-diphényl-2-propènoate ou le l'éthyl-2-cyano-3,3-diphényl-2-propènoate ;

Parmi les filtres inorganiques solaires, également appelés "écrans minéraux", que l'on peut associer à ladite composition (C₁), il y a les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être optionnellement présentés sous formes de pré-dispersions aqueuses ou huileuses.

Comme exemples de principes actifs que l'on peut associer à ladite composition (C₁), dans le cadre de la présente invention, il y a les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE^{™}MSH, le SEPICALM^{™}VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM^{™}S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM^{™}, l'ADIPOLESS^{™}, la fucoxanthine ; les protéines N-acylées ; les peptides N-acylés comme le MATRIXIL^{™} ; les acides aminés N-acylés ; les hydrolysâts partiels de protéines N-acylés ; les acides aminés ; les peptides ; les hydrolysâts totaux de protéines ; les extraits de soja, par exemple la Raffermine^{™} ; les extraits de blé par exemple la TENSINE^{™} ou la GLIADINE^{™} ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE^{™}C8G, le LIPACIDE^{™}UG, le SEPICONTROL^{™}A5 ; l'OCTOPIROX^{™} ou le SENSIVA^{™}SC50 ; les composés montrant une propriété énergisante ou stimulante comme le PHYSIOGENYL^{™}, le panthénol et ses dérivés comme le SEPICAP^{™}MP ; les actifs anti-âge comme le SEPILIFT^{™}DPHP, le LIPACIDE^{™}PVB, le SEPIVINOL^{™}, le SEPIVITAL^{™}, le MANOLIVA^{™}, le PHYTO-AGE^{™}, le TIMECODE^{™} ; le SURVICODE^{™} ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule.

Comme exemples d'agents antioxydants que l'on peut associer à ladite composition (C₁), il y a l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE^{™}GL47S (Dénomination INCI : Tetrasodium Glutamate Diacetate).

L'invention a enfin pour objet la composition (C₁) telle que définie précédemment, pour son utilisation dans une méthode de traitement thérapeutique destinée à éclaircir la peau du corps humain. Les exemples suivants illustrent l'invention sans toutefois la limiter.

### 1) - Préparation de la composition C_{1A} selon l'invention.

Le mode opératoire mis en œuvre pour préparer la composition (C_{1A}) selon l'invention est le suivant :
- Chargement d'un mélange comprenant 0,2 équivalent molaire de sorbitol, 0,6 équivalent molaire de sorbitan et 0,2 équivalent molaire d'isosorbide dans un réacteur équipé d'une agitation mécanique et d'un montage de distillation sous vide ; puis de 1,25 équivalent molaire de poudre de rhamnose monohydraté ;
- Mise sous agitation et chauffage jusqu'à la fonte totale du rhamnose ; puis sous vide partiel pour éliminer l'eau contenue dans le rhamnose ;
- Ajout du système catalytique 1,0% d'H₃PO₂ à 50% (par rapport au rhamnose) ; puis chauffage jusqu'à disparition du rhamnose ;
- Si nécessaire, neutralisation du milieu avec le mélange soude/borohydrure de sodium, en fonction de la viscosité du milieu réactionnel ;
- vidange de la composition (C_{1A}) ainsi obtenue.

### 2) - Préparation de la composition C_{1B} comparative.

Le mode opératoire du procédé décrit dans l'exemple 1 est mis en œuvre en remplaçant les 1,25 équivalents molaires de rhamnose monohydraté par 0,6 équivalent molaires de rhamnose monohydraté pour obtenir la composition (C1_{B}).

### 3) - Préparation de la composition C_{1C} comparative.

Le mode opératoire du procédé décrit dans l'exemple 1 est mis en œuvre en remplaçant le mélange comprenant 0,2 équivalents molaires de sorbitol, 0,6 équivalents molaires de sorbitan et 0,2 équivalents molaires d'isosorbide par 1 équivalent molaire de xylitol, pour obtenir la composition (C_{1C}) dont les caractéristiques analytiques sont pour obtenir la composition (C_{1C}).

Les caractéristiques analytiques des compositions (C_{1A}), (C_{1B}) et (C_{1C}) sont consignées dans le tableau 1 ci-après.

**Tableau 1**

| | **Compositions** | | |
|---|---|---|---|
| **Composants (% en masse)** | **(C_{1A})** | **(C_{1B})** | **(C_{1C})** |
| Sorbityl/sorbita n/isosorbid e polyrhamnoside | 73,8% | 49.7 % | - |
| Degré de polymérisation moyen | 1,64 | 1,30 | 1,64 |
| Sorbitan | 20,5% | 35.2 % | - |
| Isosorbide | 2,5% | 2.8% | - |
| Sorbitol | 2,8% | 1.3% | - |
| Xylityl polyrhamnoside | - | - | 74,2% |
| Xylitan | - | - | 2,2% |
| Xylitol | - | - | 22,7% |
| Eau | n.d. | n.d. | 0,3% |
| Rhamnose résiduel | 0,4% | 3.7% | 0,6% |

### 4) - Etude de l'activité éclaircissante des compositions (C_{1A}) à (C_{1C}) dans des cultures de mélanocytes B16/F1

La mise en évidence de l'activité éclaircissante a été réalisée par la mise en œuvre d'un modèle d'étude de la production de la mélanine sur une lignée de mélanocytes murins B16/F1. Ce test est couramment utilisé dans le secteur de la cosmétique et pharmaceutique.

Les mélanocytes murins de la lignée B16/F1 cultivés en monocouches, sont ensemencés dans des plaques de culture de 96 puits à la densité de 5000 cellules/puits. Les cellules sont cultivées dans un milieu de culture (milieu MCM) à 37°C, dans une atmosphère humide contenant 5 % de CO₂. Le milieu MCM a la composition suivante : Milieu DMEM (Dulbecco's Modified Eagle's Medium) contenant 4,5 g/l de glucose additionné de L-Glutamine (2 mM), de pénicilline (50 UI / ml), de streptomycine (50 µg/ml) et de sérum de veau fœtal (10% v/v). Les produits objets de l'étude sont testés à 0,01% et à 0,1% dans le milieu MCM. Les cultures de mélanocytes sont incubées en présence du produit à l'essai ou des produits de référence pendant 72 heures à 37°C, dans une atmosphère humide contenant 5 % de CO₂. Des cultures témoins sont incubées, en absence de produit, dans le milieu MCM. Ces cultures témoins sont réalisées sur chaque plaque de culture. Chaque essai est réalisé quatre fois (quatriplicate, n = 4).

### Evaluation des effets

Après 72 heures d'incubation, les milieux d'incubation des cellules (n = 4) sont prélevés et stockés à - 80°C jusqu'au moment de l'évaluation des effets. Les tapis cellulaires sont lysés dans une solution de PBS (Phosphate Buffered Saline) à l'aide d'une tige de sonication et la mélanine intracellulaire est évaluée en parallèle de la quantité de protéines. La mélanine (extracellulaire et intracellulaire) est quantifiée par spectrophotométrie à 450 nm. Une gamme d'étalonnage de mélanine est réalisée en parallèle. Les protéines sont quantifiées à l'aide d'un kit BCA (Bicinchoninic Acid Assay) et exprimées en mg/mL. Les résultats de mélanine sont exprimés en µg/ml pour la mélanine extracellulaire. Une analyse statistique à l'aide du test de Student a été effectuée afin de comparer chacune des conditions à la condition témoin.

Des essais ont été conduits lors d'une première série expérimentale avec la composition (C_{1A}) selon l'invention, comparativement au témoin de culture et les résultats obtenus sont consignés dans le Tableau 2 ci-dessous.

**Tableau 2**

| Produits testés | Mélanine intracellulaire (µg/mL) | |
|---|---|---|
| | Moyenne | Ecart-type |
| Témoin de culture | 59,3 | 1,13 |
| Acide kojique (0,005% massique) | 40,1 | 5,12 |
| Composition (C_{1A}) à 0,1% massique | 28,4 | 1,31 |
| Composition (C_{1A}) à 0,01% massique | 42,3 | 7,58 |

Des essais ont été conduits lors d'une seconde série expérimentale avec les compositions (C1_{B}) et (C1_{C}), comparativement au témoin de culture et les résultats obtenus sont consignés dans le Tableau 3 ci-dessous.

**Tableau 3**

| Produits testés | Mélanine intracellulaire (µg/mL) | |
|---|---|---|
| | Moyenne | Ecart-type |
| Témoin de culture | 32,8 | 1,62 |
| Acide kojique (0,005% massique) | 26,0 | 0,71 |
| Composition (C_{1B}) à 0,1% massique | 30,1 | 2,17 |
| Composition (C_{1B}) à 0,01% massique | 33,0 | 0,45 |
| Composition (C1_{C}) à 0,1% massique | 32,0 | 0,72 |
| Composition (C1_{C}) à 0,01% massique | 30,6 | 0,85 |

Les résultats observés permettent de mettre en évidence une inhibition significative de la production de la mélanine intracellulaire pour la composition (C_{1A}) selon l'invention. (diminution de la mélanine intracellulaire produite de 28,7% par rapport au témoin de culture pour une dose de 0,01% massique et de 52,1% par rapport au témoin de culture pour une dose de 0,01% massique).

Dans le cas de la composition (C_{1B}) il n'y a pas d'évolution de la mélanine intracellulaire produite par rapport au témoin de culture pour une dose de 0,01% massique et une diminution non significative de 8,2 % par rapport au témoin de culture pour une dose de 0,01% massique.

Dans le cas de la composition (C_{1C}) il y a des diminutions non significatives de 6,7% de la mélanine intracellulaire produite par rapport au témoin de culture pour une dose de 0,01% massique et de 2,1% de la mélanine intracellulaire produite par rapport au témoin de culture pour une dose de 0,1% massique.

Etant donné qu'une diminution de la mélanine intracellulaire produite n'est jugée « significative » que si l'on observe une diminution supérieure ou égale à 15% de la valeur de la mélanine intracellulaire produite par le témoin de culture. Il en résulte que la compositions (C_{1A}) selon l'invention permet d'éclaircir la peau humaine avec des performances améliorées et significatives.

### Exemples de formulations cosmétiques

Dans les exemples suivants les proportions sont exprimées en pourcentages pondéraux.

### Exemple 5 : Emulsion-Soin éclaircissant pour peau mature

| | |
|---|---|
| MONTANOV^{™} 202 | 2,00% |
| MONTANOV^{™} 68 | 2,00% |
| Caprylic capric triglycérides | 10,00% |
| Squalane | 10,00% |
| Eau | QSP 100% |
| Composition (C_{1A}) | 1,00% |
| SEPIGEL^{™} 305 | 0,70% |
| Magnésium ascorbyl phosphate | 2,00% |
| SEPICIDE^{™} HB | 0,30% |
| SEPICIDE^{™} CI | 0,20% |
| Parfum | 0,50% |

### Exemple 6 : Emulsion-Soin raffermissant éclaircissant

| | |
|---|---|
| MONTANOV^{™} 202 | 3,00% |
| Soude à 24 % | qs pH |
| Ethyl hexyl méthoxycinnamate | 6,00% |
| LANOL^{™}1688 | 8,00% |
| Benzophénone-3 | 4,00% |
| Eau | QSP 100% |
| Composition (C_{1A}) | 2,00% |
| SIMULGEL^{™} NS | 0,50% |
| SEPILIFT^{™} DPHP | 0,50% |
| Diméthicone | 2,00% |
| Cyclométhicone | 2,00% |
| Arbutine | 0,30% |
| SEPICIDE^{™} HB | 0,30% |
| SEPICIDE^{™} CI | 0,20 % |
| Parfum | 0,10 % |

### Exemple 7 : Gel-crème éclaircissant aux alpha-hydroxyacides

| | |
|---|---|
| Hydroxyéthylcellulose | 0,80% |
| Ethylhexyl octanoate | 5,00% |
| Sodium lactate à 60 % | 14,00% |
| Eau | QSP 100% |
| Composition (C_{1A}) | 3,00% |
| SEPIGEL^{™} 305 | 4,20% |
| SEPICIDE^{™} HB | 2,00% |
| SEPICIDE^{™} CI | 3,00% |
| Parfum | 0,10% |

### Exemple 8 : Emulsion- Soin éclaircissant

| | |
|---|---|
| MONTANOV^{™} L | 1,00% |
| Cetyl alcool | 2,00% |
| Isodecyl neopentanoate | 12,00% |
| Cetearyl octanoate | 10,00% |
| Glycérine | 3,00% |
| Eau | QSP 100 % |
| Composition (C_{1A}) | 1,00% |
| SIMULGEL^{™} EG | 2,00% |
| Acide kojique | 1,00% |
| SEPICIDE^{™} HB | 0,30% |
| SEPICIDE^{™} CI | 0,20% |
| Parfum | 0,10% |

### Exemple 9 : Lotion éclaircissante

| | |
|---|---|
| ORAMIX^{™} CG110 | 5,00% |
| KATHON^{™} CG | 0,08% |
| Eau | QSP 100% |
| Composition (C_{1A}) | 1,00% |
| Parfum | 0,10% |

Cette lotion peut être vendue en flacons ou imprégnée sur des lingettes.

Les définitions des produits commerciaux utilisés dans les exemples sont les suivantes :
SEPILIFT^{™} DPHP : (Dénomination INCI : Dipalmitoyl hydroxyproline) ;
SEPICIDE^{™} CI : Imidazoline urée, (agent conservateur) ;
SEPICIDE^{™} HB : Mélange de phénoxyéthanol, de méthylparaben, d'éthylparaben, de propyl-paraben et de butylparaben, (agent conservateur) ;
KATHON^{™} CG : (Dénomination INCI : méthyl isothiazolinone/méthyl chloroisothiazolinone) ;
SIMULGEL^{™} EG : (Dénomination INCI : Sodium acrylate/Sodium acryloyldimethyl taurate copolymer et Isohexadecane et Polysorbate 80) ;
SIMULGEL^{™} NS : (Dénomination INCI : hydroxyethylacrylate/Sodium acryloyldimethyl taurate copolymer et squalane et Polysorbate 60) ;
SEPIGEL^{™} 305 : (dénomination INCI : Polacrylamide / C13-14 Isoparaffin / Laureth-7) ;
LANOL^{™} 1688 : Ethylhexanoate de cétéaryle ;
MONTANOV^{™} L : Agent émulsionnant à base d'alcool en C14-C22 et d'alkyl polyglucoside en C12-C20 tel que ceux décrits dans la demande de brevet européen EP 0 995 487 ;
MONTANOV^{™} 202 : Agent émulsionnant à base d'alcool arachidylique d'alcool béhènylique et d'arachidyl polyglucoside ;
MONTANOV^{™} 68 est un agent émulsionnant à base d'alcool cétéarylique et cétéaryl polyglucoside
ORAMIX^{™} CG110 est un agent moussant à base d'octyl polyglucosides et de décyl polyglucosides.

## Revendications

1. Utilisation non thérapeutique d'une composition (C₁) comprenant pour 100% de sa masse :
(1) - Une quantité supérieure à 0% en masse et inférieure ou égale à 25% en masse d'un polyol de formule (I) :
HO-CH₂-(CHOH)₄-CH₂-OH (I) ;
(2) - Une quantité supérieure à 0% en masse et inférieure ou égale à 30% en masse d'un composé de formule (B₁₃) : 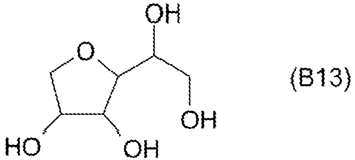
(3) - Une quantité supérieure ou égale à 0% en masse et inférieure ou égale à 5% en masse d'un composé de formule (B₁₄) : 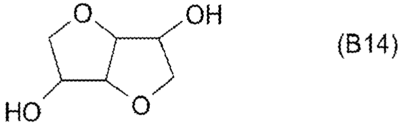
(4) - De 60% en masse à 95% en masse d'une composition (C_{R}) représentée par la formule (II) :
HO-CH₂CHOH)₄-CH₂-O-(Rham)ₓ-H (II),
formule (II) dans laquelle Rham représente le reste du rhamnose et x, qui indique le degré moyen de polymérisation dudit reste Rham, représente un nombre décimal supérieur ou égal à 1,5 et inférieur ou égal à 2,5, et
(5) - une quantité supérieure à 0% en masse et inférieure ou égale à 5% en masse de rhamnose ;
étant entendu que la somme des proportions massiques en composés ou composition selon (1), (2), (3), (4) et (5) est égale à 100% massique,
ladite utilisation étant dans le but d'éclaircir la peau humaine, et ladite utilisation étant dans une composition cosmétique.

2. Procédé non thérapeutique dans le but d'éclaircir la peau humaine comprenant au moins une étape d'application sur ladite peau humaine d'une formulation cosmétique à usage topique comprenant au moins un excipient cosmétiquement acceptable et une quantité efficace d'une composition (C₁) comprenant pour 100% de sa masse :
(1) - Une quantité supérieure à 0% en masse et inférieure ou égale à 25% en masse d'un polyol de formule (I) :
HO-CH₂-(CHOH)₄-CH₂-OH (I) ;
(2) - Une quantité supérieure à 0% en masse et inférieure ou égale à 30% en masse d'un composé de formule (B₁₃) : 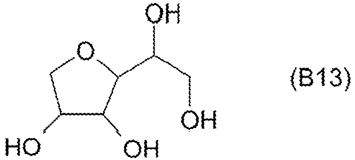
(3) - Une quantité supérieure ou égale à 0% en masse et inférieure ou égale à 5% en masse d'un composé de formule (B₁₄) : 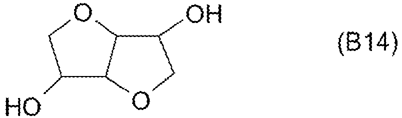
(4) - De 60% en masse à 95% en masse d'une composition (C_{R}) représentée par la formule (II) :
HO-CH₂-(CHOH)₄-CH₂-O-(Rham)ₓ-H (II),
formule (II) dans laquelle Rham représente le reste du rhamnose et x, qui indique le degré moyen de polymérisation dudit reste Rham, représente un nombre décimal supérieur ou égal à 1,5 et inférieur ou égal à 2,5, et
(5) - une quantité supérieure à 0% en masse et inférieure ou égale à 5% en masse de rhamnose ;
étant entendu que la somme des proportions massiques en composés ou composition selon (1), (2), (3), (4) et (5) est égale à 100% massique.

3. Composition (C₁) comprenant pour 100% de sa masse :
(1) - Une quantité supérieure à 0% en masse et inférieure ou égale à 25% en masse d'un polyol de formule (I) :
HO-CH₂-(CHOH)₄-CH₂-OH (I) ;
(2) - Une quantité supérieure à 0% en masse et inférieure ou égale à 30% en masse d'un composé de formule (B₁₃) : 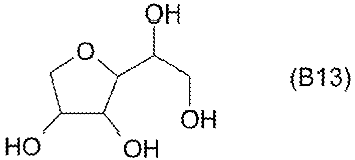
(3) - Une quantité supérieure ou égale à 0% en masse et inférieure ou égale à 5% en masse d'un composé de formule (B₁₄) : 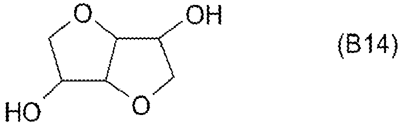
(4) - De 60% en masse à 95% en masse d'une composition (C_{R}) représentée par la formule (II) :
HO-CH₂CHOH)₄-CH₂-O-(Rham)ₓ-H (II),
formule (II) dans laquelle Rham représente le reste du rhamnose et x, qui indique le degré moyen de polymérisation dudit reste Rham, représente un nombre décimal supérieur ou égal à 1,5 et inférieur ou égal à 2,5, et
(5) - une quantité supérieure à 0% en masse et inférieure ou égale à 5% en masse de rhamnose ;
étant entendu que la somme des proportions massiques en composés ou composition selon (1), (2), (3), (4) et (5) est égale à 100% massique,
pour son utilisation dans une méthode de traitement thérapeutique destinée à éclaircir la peau du corps humain.

## Patentansprüche

1. Nicht-therapeutische Verwendung einer Zusammensetzung (C₁), die auf 100 % ihrer Masse Folgendes umfasst:
(1) - Eine Menge von mehr als 0 Massen-% und von höchstens 25 Massen-% eines Polyols der Formel (I):
HO-CH₂-(CHOH)₄-CH₂-OH (I);
(2) - Eine Menge von mehr als 0 Massen-% und von höchstens 30 Massen-% einer Verbindung der Formel (B₁₃): 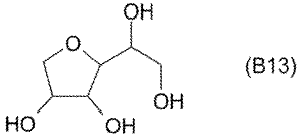
(3) - Eine Menge von mindestens 0 Massen-% und von höchstens 5 Massen-% einer Verbindung der Formel (B₁₄): 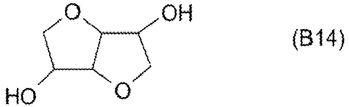
(4) - 60 Massen-% bis 95 Massen-% einer Zusammensetzung (C_{R}), welche der Darstellung in Formel (II) entspricht:
HO-CH₂-(CHOH)₄-CH₂-O-(Rham)ₓ-H (II) ,
wobei in der Formel (II) Rham für den Rest der Rhamnose steht und x, welches den mittleren Polymerisationsgrad des Restes Rham angibt, für eine Dezimalzahl von mindestens 1,5 und von höchstens 2,5 steht, und
(5) - Eine Menge von mehr als 0 Massen-% und von höchstens 5 Massen-% an Rhamnose;
wobei es sich versteht, dass die Summe der Massenanteile an Verbindungen oder Zusammensetzung gemäß (1), (2), (3), (4) und (5) gleich 100 Massen-% ist, wobei die Verwendung dazu dient, die menschliche Haut aufzuhellen, und wobei die Verwendung in einer kosmetischen Zusammensetzung erfolgt.

2. Nicht-therapeutisches Verfahren, das dazu dient, die menschliche Haut aufzuhellen, wobei es mindestens einen Schritt umfasst, in welchem auf die menschliche Haut eine kosmetische Formulierung aufgebracht wird, die zur topischen Verwendung bestimmt ist, wobei sie mindestens einen kosmetisch unbedenklichen Hilfsstoff und eine wirksame Menge einer Zusammensetzung (C₁) umfasst, welche auf 100 % ihrer Masse Folgendes umfasst:
(1) - Eine Menge von mehr als 0 Massen-% und von höchstens 25 Massen-% eines Polyols der Formel (I):
HO-CH₂-(CHOH)₄-CH₂-OH (I);
(2) - Eine Menge von mehr als 0 Massen-% und von höchstens 30 Massen-% einer Verbindung der Formel (B₁₃): 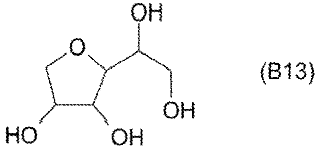
(3) - Eine Menge von mindestens 0 Massen-% und von höchstens 5 Massen-% einer Verbindung der Formel (B₁₄): 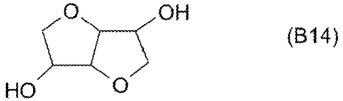
(4) - 60 Massen-% bis 95 Massen-% einer Zusammensetzung (C_{R}), welche der Darstellung in Formel (II) entspricht:
HO-CH₂-(CHOH)₄-CH₂-O-(Rham)ₓ-H (II) ,
wobei in der Formel (II) Rham für den Rest der Rhamnose steht und x, welches den mittleren Polymerisationsgrad des Restes Rham angibt, für eine Dezimalzahl von mindestens 1,5 und von höchstens 2,5 steht, und
(5) - Eine Menge von mehr als 0 Massen-% und von höchstens 5 Massen-% an Rhamnose;
wobei es sich versteht, dass die Summe der Massenanteile an Verbindungen oder Zusammensetzung gemäß (1), (2), (3), (4) und (5) gleich 100 Massen-% ist.

3. Zusammensetzung (C₁), die auf 100 % ihrer Masse Folgendes umfasst:
(1) - Eine Menge von mehr als 0 Massen-% und von höchstens 25 Massen-% eines Polyols der Formel (I):
HO-CH₂-(CHOH)₄-CH₂-OH (I);
(2) - Eine Menge von mehr als 0 Massen-% und von höchstens 30 Massen-% einer Verbindung der Formel (B₁₃): 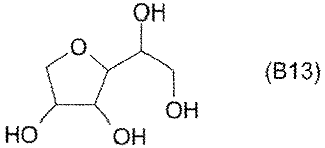
(3) - Eine Menge von mindestens 0 Massen-% und von höchstens 5 Massen-% einer Verbindung der Formel (B₁₄): 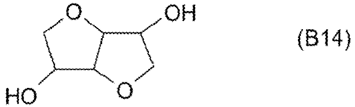
(4) - 60 Massen-% bis 95 Massen-% einer Zusammensetzung (C_{R}), welche der Darstellung in Formel (II) entspricht:
HO-CH₂-(CHOH)₄-CH₂-O-(Rham)ₓ-H (II) ,
wobei in der Formel (II) Rham für den Rest der Rhamnose steht und x, welches den mittleren Polymerisationsgrad des Restes Rham angibt, für eine Dezimalzahl von mindestens 1,5 und von höchstens 2,5 steht, und
(5) - Eine Menge von mehr als 0 Massen-% und von höchstens 5 Massen-% an Rhamnose;
wobei es sich versteht, dass die Summe der Massenanteile an Verbindungen oder Zusammensetzung gemäß (1), (2), (3), (4) und (5) gleich 100 Massen-% ist, zur Verwendung derselben in einem therapeutischen Behandlungsverfahren, welches dazu bestimmt ist, die Haut des menschlichen Körpers aufzuhellen.

## Claims

1. Non-therapeutic use of a composition (C₁) comprising per 100% of its mass:
(1) - an amount greater than 0% by mass and less than or equal to 25% by mass of a polyol of formula (I):
HO-CH₂-(CHOH)₄-CH₂-OH (I);
(2) - an amount greater than 0% by mass and less than or equal to 30% by mass of a compound of formula (B₁₃): 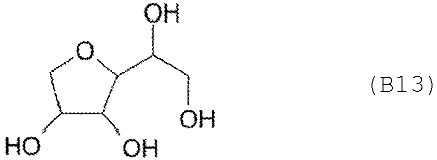
(3) - an amount greater than or equal to 0% by mass and less than or equal to 5% by mass of a compound of formula (B₁₄) : 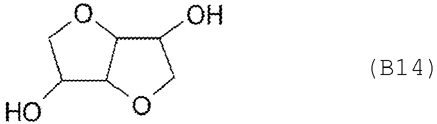
(4) - from 60% by mass to 95% by mass of a composition (C_{R}) represented by formula (II):
HO-CH₂-(CHOH)₄-CH₂-O-(Rham)ₓ-H (II),
in which formula (II) Rham represents the rhamnose residue and x, which indicates the average degree of polymerization of said Rham residue, represents a decimal number greater than or equal to 1.5 and less than or equal to 2.5, and
(5) - an amount greater than 0% by mass and less than or equal to 5% by mass of rhamnose;
with the proviso that the sum total of the proportions by mass of compounds or composition according to (1), (2), (3), (4) and (5) is equal to 100% by mass, said use being with the aim of lightening human skin and said use being in a cosmetic composition.

2. Non-therapeutic process with the aim of lightening human skin, comprising at least one step of applying to said human skin a cosmetic formulation for topical use comprising at least one cosmetically acceptable excipient and an effective amount of a composition (C₁) comprising per 100% of its mass:
(1) - an amount greater than 0% by mass and less than or equal to 25% by mass of a polyol of formula (I):
HO-CH₂-(CHOH)₄-CH₂-OH (I);
(2) - an amount greater than 0% by mass and less than or equal to 30% by mass of a compound of formula (B₁₃) : 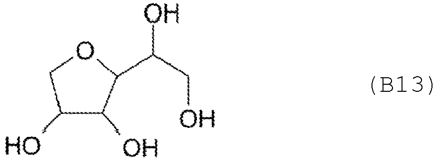
(3) - an amount greater than or equal to 0% by mass and less than or equal to 5% by mass of a compound of formula (B₁₄) : 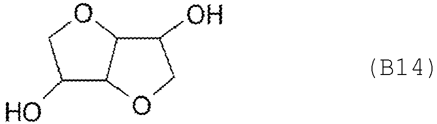
(4) - from 60% by mass to 95% by mass of a composition (C_{R}) represented by formula (II):
HO-CH₂-(CHOH)₄-CH₂-O-(Rham)ₓ-H (II),
in which formula (II) Rham represents the rhamnose residue and x, which indicates the average degree of polymerization of said Rham residue, represents a decimal number greater than or equal to 1.5 and less than or equal to 2.5, and
(5) - an amount greater than 0% by mass and less than or equal to 5% by mass of rhamnose;
with the proviso that the sum total of the proportions by mass of compounds or composition according to (1), (2), (3), (4) and (5) is equal to 100% by mass.

3. Composition (C₁) comprising per 100% of its mass:
(1) - an amount greater than 0% by mass and less than or equal to 25% by mass of a polyol of formula (I):
HO-CH₂-(CHOH)₄-CH₂-OH (I);
(2) - an amount greater than 0% by mass and less than or equal to 30% by mass of a compound of formula (B₁₃) : 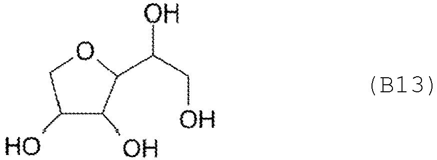
(3) - an amount greater than or equal to 0% by mass and less than or equal to 5% by mass of a compound of formula (B₁₄) : 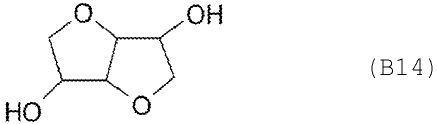
(4) - from 60% by mass to 95% by mass of a composition (C_{R}) represented by formula (II):
HO-CH₂-(CHOH)₄-CH₂-O-(Rham)ₓ-H (II),
in which formula (II) Rham represents the rhamnose residue and x, which indicates the average degree of polymerization of said Rham residue, represents a decimal number greater than or equal to 1.5 and less than or equal to 2.5, and
(5) - an amount greater than 0% by mass and less than or equal to 5% by mass of rhamnose;
with the proviso that the sum total of the proportions by mass of compounds or composition according to (1), (2), (3), (4) and (5) is equal to 100% by mass, for the use thereof in a therapeutic treatment method intended to lighten the skin of the human body.
